# EUROPEAN PATENT SPECIFICATION

(11) **EP 0 503 004 B1**
(45) Date of publication and mention of the grant of the patent: **01.05.1996**
(21) Application number: 91902537.9
(22) Date of filing: 28.11.1990
(51) Int. Cl.: A61K 7/15, A61L 9/04, B01J 13/00, C09K 3/30, C11D 17/00, A61K 7/02

(54) **POST-FOAMING SHAVING GELS**
NACHSCHÄUMENDES RASIERGEL
GELS DE RASAGE A POUVOIR MOUSSANT APRES APPLICATION

(30) Priority: 01.12.1989 GB 8927211
(43) Date of publication of application: 16.09.1992
(73) Proprietor: The Gillette Company, Boston, Massachusetts 02190 (US)
(72) Inventor: CHAUDHURI, Dwaipayan, Windsor Berkshire (GB); PARSONS, Michael, William, Berkshire (GB)
(74) Representative: Baillie, Iain Cameron
(86) International application number: US9006912
(87) International publication number: WO9107943

(56) References cited:
- GB-A- 1 444 334
- GB-A- 2 166 150
- US-A- 3 228 842
- US-A- 3 330 730
- US-A- 3 541 581
- US-A- 3 655 865
- US-A- 3 923 970
- US-A- 4 078 105
- US-A- 4 528 111

## Description

This invention is concerned with post-foaming shaving gels, that is shaving compositions which are dispensed from a container as a gel and which spontaneously foam when spread or manipulated on the skin to form a shaving foam.

Such post-foaming shaving gels are to be distinguished from ready-formed shaving foams which are dispensed from aerosol containers as a foam. Post-foaming shaving gels are described for example, in U.S. Patents 2,995,521 and 3,541,581 and in British Specifications 1,279,145, 1,444,334 and 2,166,150. They may be contained and dispensed from a collapsible metal or plastics tube, a pump dispenser, or a single compartment or a two-compartment aerosol container (the latter term being used herein generically to cover an aerosol container in which the product to be dispensed is physically separated from the propellant).

All such shaving gels contain a volatile liquid post-foaming agent. When a single compartment aerosol container is used, the post-foaming agent should be one or more of the volatile liquids used as aerosol propellants and the gel composition contains sufficient of such a propellant to obtain the desired post-foaming and is associated, in the container, with additional propellant which serves to expel the gel from the container upon actuation of the container valve. In some cases, it is necessary or desirable to provide additional pressure in the container by further introducing a compressed non-liquefied gas, such as nitrogen, in order to ensure that the whole of the container contents can be expelled.

When a two-compartment container is used, the gel (containing the required post-foaming agent) is physically separated from the propellant. The gel or the propellant may be contained in a collapsible or expandable, respectively, envelope or they may be separated by a movable piston; the gel is, of course, so positioned within the container that it can be expelled from the container outlet. A suitable expandable propellant envelope system is, for example, the GrowPak system (which progressively evolves carbon dioxide within an impermeable expandable envelope) available from Enviro-Spray N.V. Belgium.

In two-compartment containers, the propellant serves to expel the gel from the container and does not have any post-foaming function.

Whilst a number of post-foaming shaving gels are currently available on the market, we have sought to develop a shaving gel of this kind which is characterised by excellent clarity and brightness, these being features which are appealing to users and which are lacking to a greater or lesser extent in the currently available products.

We have found that these desirable characteristics can be obtained by including one or more of a selected group of polyols in a soap-based micro-emulsion gel.

According to the present invention, there is provided a post-foaming shaving gel, which comprises, by weight:

| | |
|---|---|
| 1. soap-forming fatty acid | 8.0 - 30.0% |
| 2. soap-forming base | |
| 3. light liquid paraffin | 2.0 - 6.0% |
| 4. one or more polyols comprising a polysiloxane polyether copolymer | 1.0 - 8.0% |
| 5. volatile liquid post-forming agent | 1.0 - 8.0% |
| 6. water | to 100% |

the amount of soap-forming base (2) being at least the amount required to neutralise the fatty acid (1) and the proportions of constituents (1) - (4) and (6) within the ranges specified being such that they form a stable liquid oil-in-water micro-emulsion which is converted into a stable semi-solid gel by the incorporation of the volatile liquid (5).

The invention also comprises a method of making a post-foaming shaving gel and filling it into a container from which it can be dispensed, which comprises bringing together constituents (1) - (4) and (6) specified above in proportions within the ranges specified above such that they form a stable oil-in-water micro-emulsion, introducing the micro-emulsion into the container, and simultaneously or subsequently introducing the volatile liquid (5) so that the micro-emulsion is converted into a semi-solid gel within the container.

The invention further comprises a container containing a post-forming shaving gel according to the invention.

Whilst the ranges of proportions of the constituents given above cover compositions suitable for all the types of containers referred to above, the preferred balance between constituents (1) - (4), on the one hand, and water (6), on the other, will depend on the type of container used. Thus compositions intended for pump dispensers will normally be stiffer gels than compositions intended for aerosol containers, that is the former will contain a higher proportion of constituents (1) - (4) than the latter.

The constituents of the composition according to the invention will now be described in greater detail, together with preferred proportions of the constituents for use in compositions to be dispensed from aerosol containers.

### Constituent (1).

Suitable soap-forming fatty acids are saturated or unsaturated fatty acids containing from 12 to 18 carbon atoms and include, for example, palmitic acid, stearic acid and myristic acid, and blends of two or more of these. The preferred amount of polyol (4) will depend upon the particular soap-foaming fatty acid used; the production of a clear gel requires a higher proportion of polyol (4) when stearic acid is used than when myristic acid is used.

Of soap-forming acids, palmitic acid and mixtures of palmitic acid (90-95%) and other naturally occurring fatty acids, such as stearic acid, are preferred.

The composition preferably contains from 9.0 to 11.0% of this constituent.

### Constituent (2)

Any soap-forming base can, in principle, be used as constituent (2). Suitable organic bases are, for example, triethanolamine, diethanolamine, monoethanolamine, morpholine, iso-propanolamine, aminomethyl-propanol and aminomethyl-propanediol, of which the first, triethanolamine, is the most preferred. Inorganic bases such as ammonia, sodium hydroxide and potassium hydroxide can also be used.

Mixtures of two or more of these bases can also be used, if desired.

As already indicated, the amount of base used should be at least sufficient to neutralise the fatty acid (1); it is preferred not to use an excess of the base.

### Constituent (3)

Suitable light liquid paraffins are those having a viscosity, at room temperature, of from 40 to 100 centistokes (cs). A preferred light liquid paraffin is available under the Trade Mark "Carnation" 70.

The composition preferably contains from 2.0 to 3.0% of this constituent.

### Constituent (4)

It is the use of one or more of the specified polyols which imparts excellent clarity and brightness and a desirable consistency to the gel.

Polysiloxane polyether copolymers have the CTFA name, dimethicone copolyols. They are polymeric compounds of the formula: 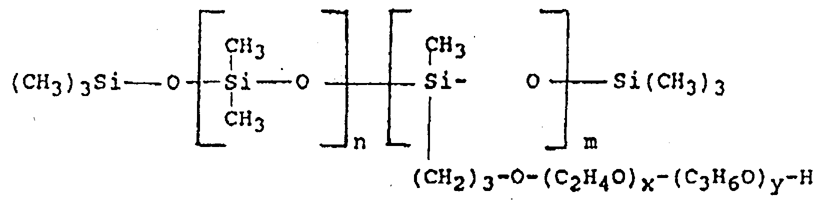
They are available from Th. Goldschmidt AG, of Essen, Germany, under the Trade Mark "Abil". Various grades are available which differ in the ratio (x/y) of ethylene oxide and propylene oxide they contain. We currently prefer the material in which the x/y ratio is 40/60; this is available as "Abil" B8863.

The polyols may additionally include 2-ethyl-1,3-hexanediol or 2-methylpentane-2,4-diol.

The composition preferably contains from 1.75 to 4.0% of this constituent.

### Constituent (5)

The post-forming agent may be any of the liquified gases or volatile liquids which are used as propellants for aerosols. It is preferred to use blends of liquid hydrocarbons and, in particular, blends of two or more of n-pentane, iso-pentane, n-butane, iso-butane and n-propane; blends of iso-pentane and iso-butane are particularly preferred.

The composition preferably contains from 2.0 to 4.0% of this constituent.

The shaving gel according to the invention may contain a number of optional ingredients including the following.

### Glycol

It is conventional to include one or more glycols in post-foaming shaving gels to control the consistency of the gel and of the foam and to give skin performance benefits. Suitable glycols are, for example, sorbitol, glycerol, propylene glycol and 1,3-butanediol, of which the first is preferred.

A suitable proportion of this constituent is 1.0 to 4.0%, more preferably 1.0 to 3.0%.

### Non-ionic surfactant

It is preferred to include up to 8.0%, more preferably 1.0 to 4.0%, of one or more non-ionic surfactants in the composition. Such surfactants improve foam quality and consistency of the gel; they also facilitate the rinsing of the razor to remove foam during the shaving operation.

It is preferred to use non-ionic surfactants which have an HLB of at least 15. Suitable surfactants meeting this requirement include for example, polyoxyethylene (POE) 20-oleyl ether (CTFA name, Oleth 20), POE 21-stearyl ether (Steareth 21), POE 100-stearyl ether (Steareth 100), 40 and 60 mole ethoxylate palm kernel oil (available under the Trade Mark "Crovol" PK 40 and PK 60), PEG 23 glyceryl laurate (available under the Trade Mark "Arlamol GM"), PEG 20 sorbitan monolaurate (available under the Trade Mark "Tween" 20), and POE 20-cetyl ether (Ceteth 20 available under the Trade Mark "Brij" 58).

### Cellulosic polymer

It is preferred to include up to 1.0%, more preferably 0.1 to 0.5%, of a cellulosic polymer in the composition. Suitable cellulosic polymers include, for example, hydroxyethyl cellulose, hydroxypropyl cellulose and hydroxypropyl methyl cellulose, of which the first, hydroxyethyl cellulose (available under the Trade Mark "Natrosol" 250 MR), is preferred.

The cellulosic polymer improves the consistency and thermal stability of the gel and enhances the lubricity of the foam. The preferred amount of such polymer depends on its molecular weight, a smaller amount (within the above range) of a high molecular weight polymer giving the same effect as a larger amount of a medium or low molecular weight polymer.

### Antioxidant

The composition preferably includes an antioxidant. Any of the antioxidants which are conventionally used in toiletry compositions may be used, butylated hydroxy toluene (BHT) and butylated hydroxyanisole (BHA) being particularly preferred.

When such an additive is used, it is preferably present in an amount up to 0.1%. Shaving aid and skin feel enhancers
The composition may include a shaving aid, that is an additive which enhances the lubricity of the composition and thus facilitates shaving, and/or one or more additives which enhance skin feel. A preferred shaving aid is, for example, polyoxyethylene. Suitable skin feel enhancers are, for example, oils, such as olive oil and peanut oil, and esters, such as isopropyl myristate and isopropyl palmitate.

Polyoxyethylene is preferably present in an amount of up to 0.1%, more preferably about 0.01%, and such oils and esters are preferably present in an amount of up to 4%, more preferably about 1%.

### Preservatives

Any of the bactericides or bacteriostats which are commonly used in toiletry formulations may be used in the shaving gel according to the invention provided that they are compatible with micro-emulsions and do not detract from the clarity of the gel. Suitable preservatives are, for example, phenoxyethanol and Quaternium 15 (CTFA) which is available under the Trade Mark "Dowicil" 200.

### Perfumes and dyestuffs

Any of the perfume and/or dyestuffs which are conventionally included in toiletry formulations for aesthetic reasons may be included in the shaving gel according to the invention provided they are compatible with it. When a perfume is used, it will be evident to the user during use and may be residual on the skin after shaving.

As regards dyestuffs, a typical composition according to the invention contains, by way of example, a combination of F, D & C Blue No. 1 at 0.00043% and F, D & C Yellow No. 10 at 0.00015%.

When the composition according to the invention is dispensed from its container, it emerges as a clear gel which converts to a foam when manipulated or spread over the skin. Completion of foaming typically takes from 30 to 60 seconds. As a foam, the composition according to the invention is an excellent aid to shaving.

The method of forming the composition according to the invention is not critical. One preferred procedure is as follows. Constituents (1), (3), (4) and the non-ionic surfactant, if present, are mixed and heated to 80° - 85°C. to give a clear oil phase, constituent (2) is added and the mixture is stirred to obtain a clear pale yellow liquid. A fifth of the total amount of constituent (6), water, which has been pre-heated to 85-90°C., is added to the previously formed mixture (the total amount of water in this context is the total amount of water required for the batch less that required to form the 2% solution of the cellulosic polymer, if present, referred to below). A thick water-in-oil emulsion is obtained which is stirred until it is uniform. The remainder of the water, also preheated, is added slowly; the emulsion slowly inverts to oil-in-water. Stirring is continued until the emulsion is homogeneous and is then discontinued.

Stirring is resumed at a speed which is sufficiently slow as to avoid aeration of the mixture. The latter is cooled and the glycol, if present, is added. At 60°C., a pre-formed 2% aqueous solution of the cellulosic polymer, if present, is added, the water of this solution completing the water content of the mixture. Dyes, if used, are added at this point and cooling and slow mixing continued. At 40°C., perfume, if used, is added and slow mixing is continued until the batch is clear. Cooling is continued to ambient temperature. The composition is then ready for filling into the containers from which, after addition of constituent (5), the post-foaming agent, it will be dispensed.

In order that the invention may be more fully understood, the following examples, in which all proportions are by weight, are given by way of illustration:

### Examples 1-4 (Example 1 and 4 are comparative)

Shaving gels of the following compositions were formed.

| Example | 1 | 2 | 3 | 4 |
|---|---|---|---|---|
| Palmitic acid | 9.69 | 9.69 | 9.69 | 9.69 |
| Triethanolamine | 5.69 | 5.69 | 5.69 | 5.69 |
| Liquid paraffin | 2.90 | 2.90 | 2.90 | 2.90 |
| 2-Ethyl-1,3-hexanediol | 1.94 | 1.45 | - | - |
| Abil B8863 | - | 1.45 | 3.00 | - |
| 2-Methylpentane-2,4-diol | - | - | - | 3.0 |
| Glycerol | 1.94 | - | - | - |
| Oleth 20 | 1.94 | 1.94 | 1.94 | 1.94 |
| Natrosol 250MR | 0.39 | 0.39 | 0.39 | 0.39 |
| Perfume | q.s. | q.s. | q.s. | q.s. |
| Dyes (green) | q.s. | q.s. | q.s. | q.s. |
| Iso-pentane | 2.40 | 2.40 | 2.40 | 2.40 |
| Iso-butane | 0.80 | 0.80 | 0.80 | 0.80 |
| Water | ------to 100% --------- | | | |

In each case, all the constituents, apart from the post-foaming agents (5), were mixed to form a clear green micro-emulsion following the preferred procedure described above. On completion of cooling to 25-30°C., the liquid composition was filled into the upper part of a two-compartment aerosol container and the valve was crimped. The iso-pentane/iso-butane mixture was added through the valve and the container was shaken to disperse the liquid hydrocarbons.

The propellant was introduced into the lower compartment through a hole in the base of the container which was then sealed with a plug.

After standing for 72 hours to allow for stabilisation of the gel, the container was ready for use.

Upon actuation of the container valve, the composition was dispensed as a clear and bright green gel which gave a lubricious shaving foam when spread over the skin.

## Claims

1. A post-foaming shaving gel, which comprises, by weight:
| | |
|---|---|
| 1. soap-forming fatty acid | 8.0 - 30.0% |
| 2. soap-forming base | |
| 3. light liquid paraffin | 2.0 - 6.0% |
| 4. one or more polyols comprising a polysiloxane polyether copolymer | 1.0 - 8.0% |
| 5. volatile liquid post-forming agent | 1.0 - 8.0% |
| 6. water | to 100% |
the amount of soap-forming base (2) being at least the amount required to neutralise the fatty acid (1) and the proportions of constituents (1) - (4) and (6) within the ranges specified being such that they form a stable liquid oil-in-water micro-emulsion which is converted into a stable semi-solid gel by the incorporation of the volatile liquid (5).

2. A shaving gel according to claim 1, in which constituent (1) is palmitic acid, and/or constituent (2) is triethanolamine, and/or constituent (5) it a blend of two or more of n-pentane, iso-pentane, n-butane, iso-butane, and n-propane.

3. A shaving gel according to claim 1 or 2, which comprises from 9.0 to 11.0% of constituent (1).

4. A shaving gel according to any of claims 1 to 3, which comprises from 2.0 to 3.0% of constituent (3), and/or from 1.75 to 4.0% of constituent (4).

5. A shaving gel according to any of claims 1 to 4, which comprises from 2.0 to 4.0% of constituent (5).

6. A shaving gel according to any of claims 1 to 5, which additionally comprises one or more of the following:
(a) from 1.0 to 4.0% of one or more glycols;
(b) up to 8.0% of one or more non-ionic surfactants which have an HLB of at least 15;
(c) up to 1.0% of a cellulosic polymer; and
(d) one or more antioxidants, shaving aids, skin feel enhancers, preservatives, perfumes and/or dyestuffs.

7. A shaving gel according to claim 6, in which the glycol is sorbitol, glycerol, propylene glycol or 1,3-butanediol.

8. A shaving gel according to claim 6, which comprises from 1.0 to 4.0% of said surfactant(s).

9. A shaving gel according to claim 6, in which the cellulosic polymer is hydroxyethyl cellulose, hydroxypropyl cellulose or hydroxypropyl methyl cellulose.

10. A method of making a post-foaming shaving gel and filling it into a container from which it can be dispensed, which comprises bringing together constituents (1) - (4) and (6) specified in claim 1 in proportions within the ranges specified therein, and, if desired, any one or more of the optional constituents specified in claim 6, such that they form a stable oil-in-water micro-emulsion, introducing the micro-emulsion into the container, and simultaneously or subsequently introducing the volatile liquid (5) so that the micro-emulsion is converted into a semi-solid gel within the container.

## Patentansprüche

1. Nachschäumendes Rasiergel, umfassend in Gewichtsprozent:
| | |
|---|---|
| (1) seifenbildende Fettsäure | 8,0...30,0 |
| (2) seifenbildende Base | |
| (3) leichtes, flüssiges Paraffin | 2,0... 6,0 |
| (4) ein oder mehrere Polyole, umfassend ein Polysiloxan/Polyether-Copolymer | 1,0... 8,0 |
| (5) flüchtiges, flüssiges Mittel zum Nachschäumen | 1,0... 8,0 |
| (6) Wasser | bis 100 |
wobei die Menge der seifenbildenden Base (2) mindestens die Menge ist, die zum Neutralisieren der Fettsäure (1) erforderlich ist, und wobei die Anteile der Bestandteile (1) ... (4) sowie (6) innerhalb der angegebenen Bereiche so liegen, so daß sie eine stabile, flüssige Öl-in-Wasser-Mikroemulsion bilden, die durch Einmischen der flüchtigen Flüssigkeit (5) in ein stabiles, halbfestes Gel überführt wird.

2. Rasiergel nach Anspruch 1, worin Bestandteil (1) Palmitinsäure, und/oder Bestandteil (2) Triethanolamin, und/oder Bestandteil (5) ein Blend von zwei oder mehren von n-Pentan, Isopentan, n-Butan, Isobutan und n-Propan ist.

3. Rasiergel nach Anspruch 1 oder 2, umfassend 9,0...11,0 % Bestandteil (1).

4. Rasiergel nach Anspruch 1 bis 3, umfassend 2,0...3,0 % Bestandteil (3), und/oder 1,75...4,0 % Bestandteil (4).

5. Rasiergel nach Anspruch 1 bis 4, umfassend 2,0...4,0 % Bestandteil (5).

6. Rasiergel nach Anspruch 1 bis 5, umfassend zusätzlich ein/oder mehrere der folgenden:
(a) 1,0 ...4,0 % ein oder mehrere Glykole;
(b) bis zu 8,0 % ein oder mehrere nichtionische Tenside mit einem HLB-Wert von mindestens 15;
(c) bis zu 1,0 % Cellulose-Polymer; sowie
(d) ein oder mehrere Antioxidantien, Rasierhilfsmittel, hautpflegendes Mittel, keimhemmende Mittel, Parfüms und/oder Farbstoffe.

7. Rasiergel nach Anspruch 6, worin das Glykol Sorbitol, Glycerin, Propylenglykol oder 1,3-Butandiol ist.

8. Rasiergel nach Anspruch 6, umfassend 1,0.....4,0 % des/der Tensid/Tenside.

9. Rasiergel nach Anspruch 6, worin das Cellulose-Polymer Hydroxyethylcellulose, Hydroxypropylcellulose oder Hydroxypropylmethylcellulose ist.

10. Verfahren zum Erzeugen eines nachschäumenden Rasiergels und zu seinem Einfülen in einen Behälter, aus dem es gespendet werden kann, welches Verfahren umfaßt:
Zusammenbringen der in Anspruch 1 angegebenen Bestandteile (1)...(4) und (6), deren Anteile innerhalb der dort angegebenen Bereichen liegen; und, sofern angestrebt, einen oder mehrere der in Anspruch 6 angegebenen wahlweisen Bestandteile derart, daß sie eine stabile Öl-in-Wasser-Mikroemulsion bilden;
Einfüllen der Mikroemulsion in den Behälter; und
gleichzeitiges oder aufeinanderfolgendes Einfüllen der flüchtigen Flüssigkeit (5), so daß die Mikroemulsion im Inneren des Behälters in ein halbfestes Gel überführt wird.

## Revendications

1. Gel de rasage à post-moussage qui comprend, en masse :
| | |
|---|---|
| 1. acide gras formateur de savon | 8,0-30,0% |
| 2. base formatrice de savon | |
| 3. paraffine liquide légère | 2,0-6,0% |
| 4. un ou plusieurs polyols comprenant un copolymère polysiloxane-polyéther | 1,0-8,0% |
| 5. agent de post-moussage liquide volatil | 1,0-8,0% |
| 6. eau | pour 100% |
la quantité de base formatrice de savon (2) étant au moins la quantité nécessaire pour neutraliser l'acide gras (1) et les proportions des constituants (1)-(4) et (6) dans les domaines spécifiés étant telles qu'ils forment une micro-émulsion huile dans l'eau liquide stable qui est convertie en un gel semi-solide stable par incorporation du liquide volatil (5).

2. Gel de rasage selon la revendication 1, dans lequel le constituant (1) et l'acide palmitique et/ou le constituant (2) et la triéthylamine et/ou le constituant (5) est un mélange de deux ou plusieurs substances parmi le n-pentane, l'isopentane, le n-butane, l'isobutane et le n-propane.

3. Gel de rasage selon la revendication 1 ou 2, qui comprend de 9,0 à 11,0% de constituant (1).

4. Gel de rasage selon l'une quelconque des revendications 1 à 3, qui comprend de 2,0 à 3,0% de constituant (3) et/ou de 1,75 à 4,0% de constituant (4).

5. Gel de rasage selon l'une quelconque des revendications 1 à 4, qui comprend de 2,0 à 4,0% de constituant (5).

6. Gel de rasage selon l'une quelconque des revendications 1 à 5, qui comprend en outre un ou plusieurs des constituants suivants :
(a) de 1,0 à 4,0% d'un ou plusieurs glycols,
(b)jusqu'à 8,0% d'un ou plusieurs tensioactifs non ioniques qui ont une HLB d'au moins 15,
(c) jusqu'à 1,0% d'un polymère cellulosique, et
(d) un ou plusieurs antioxydants, adjuvants de rasage, agents améliorant la sensation cutanée, conservateurs, parfums et/ou colorants.

7. Gel de rasage selon la revendication 6, dans lequel le glycol est le sorbitol, le glycérol, le propylèneglycol ou le 1,3-butanediol.

8. Gel de rasage selon la revendication 6, qui comprend de 1,0 à 4,0% dudit ou desdits tensioactifs.

9. Gel de rasage selon la revendication 6, dans lequel le polymère cellulosique est l'hydroxyéthylcellulose, l'hydroxypropylcellulose ou l'hydroxypropylméthylcellulose.

10. Procédé pour former un gel de rasage à post-moussage et pour l'introduire dans un récipient depuis lequel il peut être distribué, qui comprend la combinaison des constituants (1)-(4) et (6) spécifiés dans la revendication 1 dans des proportions situées dans les domaines spécifiés ici, et, si on le souhaite, d'un quelconque ou de plusieurs quelconques des constituants facultatifs spécifiés dans la revendication 6 de manière qu'ils forment une micro-émulsion (8) huile dans l'eau stable, l'introduction de la micro-émulsion dans le récipient et, simultanément ou ensuite, l'introduction du liquide volatile (5) de manière que la micro-émulsion soit convertie en un gel semi-solide à l'intérieur du récipient.
